# EUROPÄISCHE PATENTSCHRIFT

(11) **EP 0 985 387 B1**
(45) Veröffentlichungstag und Bekanntmachung des Hinweises auf die Patenterteilung: **20.02.2002**
(21) Anmeldenummer: 98122022.1
(22) Anmeldetag: 20.11.1998
(51) Int. Cl.: A61F 2/46, A61F 2/30

(54) **Bausatz zum Implantieren einer zementfixierbaren Endoprothese**
Kit for implanting a cementable endoprosthesis
Jeu de pièces pour implanter une endoprothèse cimentable

(30) Priorität: 10.09.1998 EP 98810897
(43) Veröffentlichungstag der Anmeldung: 15.03.2000
(60) Teilanmeldung: 01118202.9
(73) Patentinhaber: PLUS ENDOPROTHETIK AG, 6343 Rotkreuz (CH)
(72) Erfinder: Schmotzer, Hans Dr., 5742 Kölliken (CH); Hässig, Christoph, 5024 Küttigen (CH)
(74) Vertreter: Patentanwälte Schaad, Balass, Menzl & Partner AG

(56) Entgegenhaltungen:
- EP-A- 0 266 081
- DE-A- 19 518 391
- GB-A- 2 104 390
- US-A- 4 337 773
- US-A- 4 827 919
- US-A- 4 997 448
- US-A- 5 047 061
- US-A- 5 755 793
- US-A- 5 766 262
- US-A- 5 792 143

## Beschreibung

Die Erfindung betrifft einen Bausatz zum Implantieren einer zementfixierbaren Endoprothese gemäss dem Oberbegriff von Anspruch 1.

Aus der Druckschrift DE 195 18 391 A1 ist ein proximales Zentrier- und Dichtelement zur Implantation eines zementfixierbaren Endoprothesenschaftes bekannt, welches einerseits als proximale Zentrierhilfe dient und andererseits ein Entweichen des Zementes nach proximal verhindert, wodurch beim Einführen des Endoprothesenschaftes in den Markraum eine Druckerhöhung des sich darin befindlichen Knochenzementes resultiert.

Dieses bekannte Kombination von Endoprothesenschaft sowie angepasst ausgestaltetem Zentrier- und Dichtelement weist den Nachteil auf, dass die Einführtiefe des Endoprothesenschaft in den Markraum des Femurs in proximal-distaler Richtung nur sehr schlecht und daher ungenau einstellbar ist. Zudem weist das Zentrier- und Dichtelement eine nur beschränkte Dichtwirkung sowie eine ungenügende Zentrierung in medial-lateraler Richtung auf.

Es ist Aufgabe der vorliegenden Erfindung ein Instrumentarium zu schaffen, welches ein präziseres Implantieren eines zementfixierbaren Endoprothesenschaftes erlaubt.

Diese Aufgabe wird gelöst mit einem Bausatz aufweisend die Merkmale von Anspruch 1. Die Unteransprüche 2 bis 17 beziehen sich auf weitere, vorteilhafte Ausgestaltungen dieses Bausatzes.

Die Aufgabe wird insbesondere gelöst mit einem Bausatz zum Implantieren einer zementfixierbaren Endoprothese, umfassend ein Setzinstrument sowie zumindest zwei zu implantierende Komponenten, einen Endoprothesenschaft und ein proximales Zentrier- und / oder Dichtelement, wobei der Schaft sowie das Setzinstrument gegenseitig ankoppelbar ausgestaltet sind, und wobei das Zentrier- und / oder Dichtelement am Schaft anlegbar und in dessen Verlaufsrichtung verschiebbar ausgestaltet ist, und wobei entweder das Setzinstrument ein Anschlagteil umfasst, welches einen Anschlag bezüglich dem Zentrier- und / oder Dichtelement ausbildet, oder am Setzinstrument beziehungsweise am Schaft eine Markierung angebracht ist, um eine definierte gegenseitige Lage zwischen dem Zentrier- und / oder Dichtelement sowie dem Schaft zu gewährleisten.

Diese erfindungsgemässe Ausgestaltung weist den Vorteil auf, dass die Setztiefe des Endoprothesenschaftes im Markraum des Femurs noch während dem Implantieren einstellbar ist. Daher ist es noch während dem Implantieren möglich beispielsweise die Länge des Oberschenkels bzw. die Setztiefe des Endoprothesenschaftes derart einzustellen, dass nach erfolgter Operation beide Beine dieselbe Länge aufweisen. In einer bevorzugten Ausgestaltung umfasse der Bausatz ein Manipulierinstrument, welches vor dem Setzen des Schaftes in den Markraum des Femurs eingeführt wird, um die optimale Setztiefe zu ermitteln, so dass insbesondere eine optimale Beinlänge oder eine optimale Bänderspannung erreicht wird. Die derart ermittelte, optimale Setztiefe wird abgelesen und daraufhin der Endoprothesenschaft entsprechend dieser Setztiefe in den Femur eingeführt.

Dem proximalen Zentrier- und / oder Dichtelement kommt im proximalen Bereich des Femurs die Aufgabe zu, den Endoprothesenschaft im Markraum zu zentrieren oder den Spalt zwischen Markraum und Schaft abzudichten, um den im Markraum befindlichen Knochenzement vor dem Ausfliessen zu hindern oder gleichzeitig sowohl zu zentrieren als auch abzudichten. Dieses sowohl zentrierende als auch dichtende Zentrier- und Dichtelement ist bevorzugt derart der Geometrie des Schaftes angepasst ausgestaltet, dass ein Verschieben des Schaftes entlang des Dichtelementes in distaler Richtung unter Beibehaltung der Dichtwirkung möglich ist.

Das Zentrier- und Dichtelement ist am resezierten Schenkelhals anlegbar und in dessen proximalem Abschnitt zumindest teilweise in den Markraum einführbar, so dass der daraufhin einzuführende Endoprothesenschaft allseitig zentriert im Markraum eingeführt wird, wobei das Element zudem eine Dichtwirkung ausübt, so dass der sich im Markraum befindliche Knochenzement am Austritt behindert wird.

Das proximale Zentrier- und Dichtelement besteht in einer vorteilhaften Ausgestaltung aus einem hülsenförmigen, sich in einer proximal-distalen Richtung erstreckenden Körper, wobei der Körper zwei breitseitige Begrenzungen sowie zwei schmalseitige Begrenzungen umfasst, welche einen im wesentlichen rechteckförmigen Innenraum umschliessen, und wobei die zwei breitseitigen Begrenzungen je eine Innenseitenfläche ausbilden, welche in proximal-distaler Richtung im wesentlichen parallel verlaufen. Dieses Zentrier- und Dichtelement ist besonders geeignet für blattförmig ausgestaltete Endoprothesenschäfte, deren Breitseiten im proximal-distaler Richtung etwa parallel verlaufen. Dadurch wird zwischen dem Zentrier- und Dichtelement sowie dem Endoprothesenschaft eine besonders hohe Dichtwirkung erzielt. Ein Zentrier- und / oder Dichtelement ist als hülsenförmiger Körper ausgestaltet, dessen Innenraum in einer vorteilhaften Ausführungsform derart ausgebildet ist, dass auch beim Implantieren des Endoprothesenschaftes eine Bewegung des Schaftes in proximal-distaler Richtung möglich ist. Sollte dies erwünscht sein, so könnte der Schaft sich auch noch längere Zeit nach dem Implantieren setzen, da die Bewegung in proximal-distaler Richtung nicht behindert wird.

Das Zentrier- und / oder Dichtelement besteht vorteilhafterweise aus einem polymerisierten Knochenzement, insbesondere aus Polymethylmetacrylat (PMMA). Beim Implantieren verbindet sich dieses Zentrier- und / oder Dichtelement chemisch mit dem vorhandenen Knochenzement zu einer insbesondere homogenen Verbindung. Das Zentrier- und / oder Dichtelement kann jedoch auch aus einem andere Werkstoff, insbesondere aus einem metallischen Werkstoff wie einer biokompatiblen Titanlegierung gefertigt sein.

Das Zentrier- und / oder Dichtelement erfüllt entweder eine zentrierende oder eine dichtende Funktion, oder beide Funktionen gleichzeitig, welche sind:
- Zentrieren des Schaftes im proximalen Abschnitt des Markraumes;
- Zentrierte Führungen des Schaftes in proximal-distaler Richtung während dem Setzen des Schaftes ;
- Verhinderung eines Kippens in medial-lateraler Richtung sowie eines Verdrehens des Schaftes;
- Abdichten des sich zwischen dem Schaft und dem Femur im proximalen Bereich ergebenden Spaltes, so dass auf den sich im Markraum befindenden Knochenzement ein Druck ausgeübt wird.

Das Zentrier- und / oder Dichtelement weist in proximaler Richtung eine Abschlussfläche auf, welche vorteilhafterweise als Referenzfläche verwendet wird. Vorzugsweise wird das Zentrier- und / oder Dichtelement derart in den Markraum eingeführt, dass diese Referenzfläche bündig zur resezierten Fläche des Schenkelhalses verläuft. Der Endoprothesenschaft wird vor dem Einführen an einem Setzinstrument befestigt, wobei am Setzinstrument zudem ein Distanzelement befestigt ist, welches derart Ausgestaltet ist, dass es bei tief in den Markraum eingeführtem Endoprothesenschaft auf der Referenzfläche des Zentrier- und / oder Dichtelementes aufliegt und ein weiteres Einführen des Schaftes. verhindert. Durch dieses Distanzelement, das in verschiendenen Grössen verfügbar ist, kann die Setztiefe des Endoprothesenschaftes bezüglich der Resektionsfläche genau eingestellt werden. Entsprechend der gewünschten Setztiefe wird ein entsprechendes Distanzelement ausgewählt und am Setzinstrument vor dem Einführen des Endoprothesenschaftes befestigt.

Unter einem Bausatz werden die gegenseitig angepasst ausgestalteten Teile Zentrier- und / oder Dichtelement, Endoprothesenschaft sowie Setzinstrument, gegebenenfalls in Kombination mit einem oder mehreren Distanzelementen verstanden, wobei von diesem Bausatz nur das Zentrier- und / oder Dichtelement sowie der Endoprothesenschaft als Implantat zum Verbleiben im Körper bestimmt sind.

Die Erfindung wird nachfolgend mit Hilfe von Figuren an mehreren Ausführungsbeispielen Beschreiben. Es zeigen:
- Fig. 1: eine perspektivische Ansicht eines proximalen Zentrier- und / oder Dichtelementes;
- Fig. 2a: eine Seitenansicht eines weiteren proximalen Zentrier- und / oder Dichtelementes;
- Fig. 2b: einen Querschnitt durch das Element gemäss Fig. 2a;
- Fig. 2c: eine Aufsicht auf das Element gemäss Fig. 2a;
- Fig. 3: ein Bausatz umfassend ein Setzinstrument, ein Zentrier- und / oder Dichtelement, ein Distanzelement sowie einen Schaft;
- Fig. 4: ein mit einem Setzinstrument gemäss Fig. 3 eingeführter Schaft;
- Fig. 5: ein Querschnitt durch eine weiteres Ausführungsbeispiel eines Zentrier- und / oder Dichtelementes;
- Fig. 6: ein weiterer Bausatz umfassend ein Setzinstrument, ein Zentrier- und / oder Dichtelement sowie einen Schaft;
- Fig. 7: ein weiterer Bausatz umfassend ein Setzinstrument, ein Zentrier- und / oder Dichtelement sowie einen Schaft.

Fig. 3 zeigt eine Ausführungsbeispiel eines erfindungsgemässen Bausatzes, umfassend einen Endoprothesenschaft 4, welcher als Geradschaft ausgestaltet ist, wobei der eigentliche Schaft 4f einen im wesentlichen rechteckigen Querschnitt mit zwei schmalseitigen Seitenflächen 4c,4d sowie zwei breitseitigen Seitenflächen 4e aufweist. Am proximalen Ende weist der Schaft 4f ein Innengewinde 4a sowie einen Konus 4b für einen Gelenkkopf auf. Der Bausatz umfasst weiter ein Setzinstrument 3, welches eine Verbindungsstange 3d aufweist, an welcher ein Stempel 3c befestigt ist, welcher in ein Adapterteil 3b sowie ein Aussengewinde 3a mündet. Der Bausatz umfasst weiter ein Distanzelement 2, welches als hülsenförmiger, rechteckiger Körper 2d ausgestaltet ist, mit zwei Seitenflügeln 2a, welche eine obere Anschlagfläche 2b sowie eine untere Anschlagfläche 2c ausbilden. Weiter umfasst der Bausatz ein proximales Zentrier- und / oder Dichtelement 1. Das Distanzelement 2 ist derart ausgestaltet, dass es mit dessen oberen Anschlagfläche 2b am Stempel 3c anliegt. Beim Einführen des Endoprothesenschaftes 4 kommt die untere Anschlagfläche 2c irgendwann auf der Referenzfläche 1m, 1n des proximalen Zentrier- und / oder Dichtelementes 1 zu liegen und begrenzt dadurch in Verlaufsrichtung des Schaftes die gegenseitige Lage von Endoprothesenschaft 4 und Zentrier- und / oder Dichtelement 1. Da das Zentrier- und / oder Dichtelement 1 während dem Implantieren mit dessen Fläche 1m, 1n vorzugsweise bündig zur Resektionsfläche des Femurs liegt wird durch das Distanzelement 2 die Setztiefe des Schaftes 4f bestimmt. Einem Operateur steht eine Auswahl von Distanzelementen 2 zur Verfügung, welche in proximal-distaler Richtung C unterschiedlich lang ausgestaltet sind. Das in Fig. 3 und 4 dargestellte Distanzelement 2 ist nur als ein Ausführungsbeispiel aus einer Vielzahl von Ausführungsmöglichkeiten zu betrachten. Die Aufgabe des Distanzelementes 2 ist einen oberen sowie einen unteren Anschlag 2b,2c auszubilden, um eine definierte Setztiefe des Schaftes 4 bezüglich dem proximalen Zentrier- und / oder Dichtelement 1, bzw. dessen Referenzfläche 1m, 1n zu gewährleisten. Diese Funktion kann mit unterschiedlichst ausgestalteten Distanzelementen 2 erfüllt werden.

Fig. 4 zeigt einen Endoprothesenschaft 4 in eingeführter Lage, wobei der Femur nicht dargestellt ist. Nach dem Einführen eines Manipulierschaftes in den Markraum bestimmt der Operateur die Einführtiefe des Schaftes bezüglich der Resektionsebene. Aus der Mehrzahl von Distanzelementen wählt der Operateur dasjenige aus, welche die beabsichtige Einführtiefe gewährleistet. Diese ausgewählte Distanzelement 2 wird am Stempel 3c des Setzinstrumentes 3 befestigt und danach der Endoprothesenschaft 4 über das Innengewinde 4a fest am Aussengewinde 3a angeschraubt. Das Zentrier- und / oder Dichtelement 1 wird daraufhin von distaler Richtung her auf den Schaft 4f aufgeschoben. Daraufhin wird der Endoprothesenschaft 4 zusammen mit dem Zentrier- und / oder Dichtelement 1, wie in Fig. 4 dargestellt, bis zum Anschlag des Distanzelementes 2 mit dem proximalen Zentrier- und / oder Dichtelement 1 sowie bis zur Resektionsebene, welche eine Referenzebene bildet, in den Markraum eingeführt. Dabei wird der sich im Markraum befindliche Knochenzement komprimiert und gegen das Zentrier- und / oder Dichtelement 1 hin aus dem Markraum gedrückt. Zumindest die Innenseitenflächen 1b, 1c, 1d liegen am Endoprothesenschaft 4 an und bewirken eine dichtende Wirkung. Sofern der Endoprothesenschaft 4 mit dessen Seitenfläche 4d an der Innenseitenfläche 1e nicht anliegt bildet sich zwischen diesen Flächen ein Spalt 1p durch welchen der Knochenzement entweichen kann. Ein Operateur kann diesen Spalt 1p beispielsweise mit einem Finger abdecken und dadurch das Entweichen des Knochenzementes durch Anpressen des Fingers an den Spalt 1p oder durch entsprechendes Loslassen relativ genau kontrollieren. Die Innenseitenfläche 1e kann auch derart bezüglich der Seitenfläche 4d angeordnet sein oder der Schaft 4 derart tief in das Zentrier- und/oder Dichtelement 1 gestossen werden, dass zwischen diesen beiden Flächen 1e,4d eine dichtende Wirkung entsteht, so dass das Element 1 gleichzeitig als Zentrier- und Dichtelement wirkt. In einer bevorzugten Ausführungsform ist das Zentrier- und / oder Dichtelement 1 derart ausgestaltet und der Knochenzement derart gewählt, dass dieser das proximale Zentrier- und / oder Dichtelement allseitig umspühlt, in dem der Knochenzement sowohl zwischen den Innenseitenflächen 1b,1c,1d,1e und dem Schaft 4a als auch zwischen der Aussenfläche des Zentrier- und / oder Dichtelementes 1 und dem Femur ausgepresst wird.

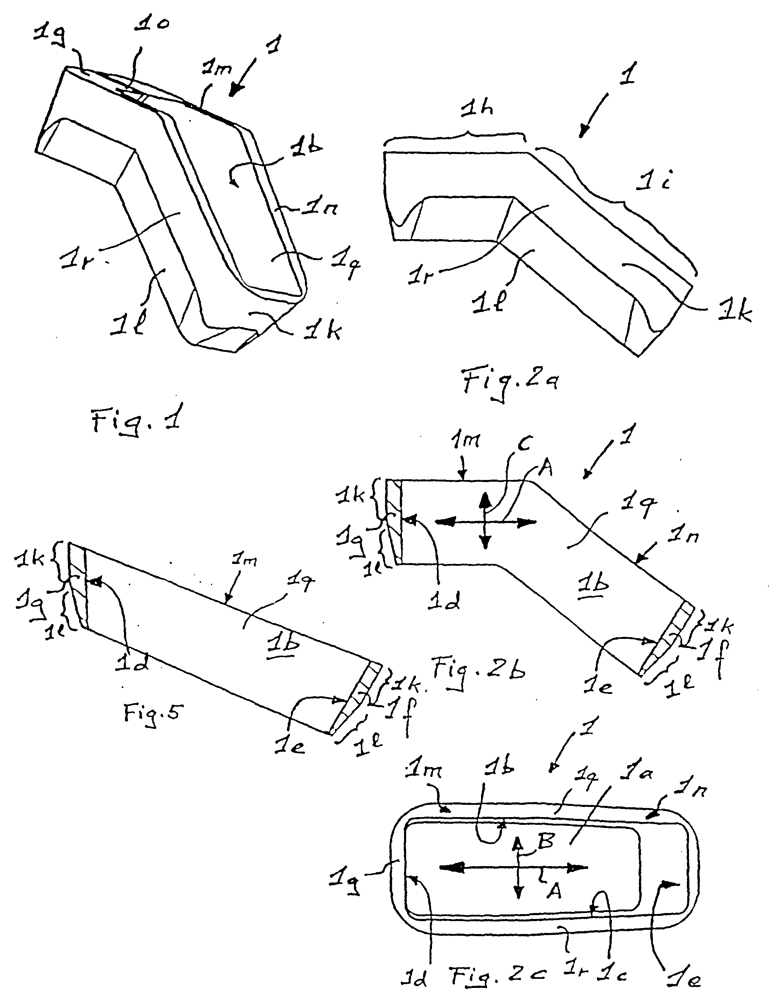

Ein Ausführungsbeispiel des Zentrier- und / oder Dichtelementes wird an Hand der Fig. 2a bis Fig. 2c beschrieben. Das Zentrier- und / oder Dichtelement besteht aus einem hülsenförmigen, sich in einer proximal-distalen Richtung C erstreckenden Körper, welcher zwei breitseitige Begrenzungen 1q,1r sowie zwei schmalseitige Begrenzungen 1f,1g aufweist, welche, wie aus der Aufsicht gemäss Fig. 2c ersichtlich, einen im wesentlichen rechteckförmigen Innenraum 1a umschliessen. Die Innenseitenflächen 1b,1c der breitseitigen Begrenzungen 1q,1r sind im wesentlichen parallel zur proximal-distalen Richtung C als auch parallel zur lateral-medialen Richtung A verlaufend ausgestaltet. Im dargestellten Ausführungsbeispiel verläuft die Innenseitenfläche 1d der schmalseitigen Begrenzung 1g, wie aus Fig. 2c ersichtlich, parallel zur proximal-distalen Richtung C. Die breitseitigen Innenseitenflächen 1b,1c könnten ebenfalls parallel zur proximal-distalen Richtung C verlaufend ausgestaltet sein, weisen jedoch im dargestellten Ausführungsbeispiel einen in distaler Richtung leicht konvergierenden Verlauf auf, was den Vorteil einer verbesserten Dichtwirkung zwischen Endoprothesenschaft 4 und Innenseitenfläche 1b, 1c ergibt. Die zweite schmalseitige Begrenzung 1f weist eine gegenüber der Innenseitenfläche 1d geneigt verlaufende Innenseitenfläche 1e auf.

Im dargestellten Ausführungsbeispiel weisen alle Begrenzungen 1q,1r,1f,1g einen sich in proximal-distaler Richtung C erstreckender Teilabschnitt 11 auf, welcher anschliessend an den Teilabschnitt 1k eine sich zur distalen Richtung hin verjüngende Wandstärke ausbildet, wie dies insbesondere aus dem in Fig. 2b dargestellten Querschnitt ersichtlich ist. In proximaler Richtung enden die breitseitigen Begrenzungen 1q,1r in einer Referenzfläche 1m,1n. Diese in medial-lateraler Richtung A verlaufende Referenzfläche 1m,1n weist, wie aus Fig. 2a und 2b ersichtlich, einen dem Verlauf der breitseitigen Begrenzung 1r folgenden, geknickten Verlauf auf.

Im Unterschied zum Ausführungsbeispiel gemäss den Figuren 2a bis 2c weist der in Fig. 5 dargestellte, ansonst gleich ausgestaltete Körper 1, zwei geradlinig verlaufende, d.h. keine Knickstelle aufweisende, breitseitigen Begrenzungen 1r,1q auf.

Im Unterschied zum Ausführungsbeispiel gemäss den Figuren 2a bis 2c weist der in Fig. 1 dargestellte Körper 1 ein in medial-lateraler Richtung A breiter ausgestaltete schmalseitige Begrenzung 1g auf, welche zudem in der Mitte einen in proximal-distaler Richtung C verlaufenden, durchgehenden Spalt 1o aufweist.

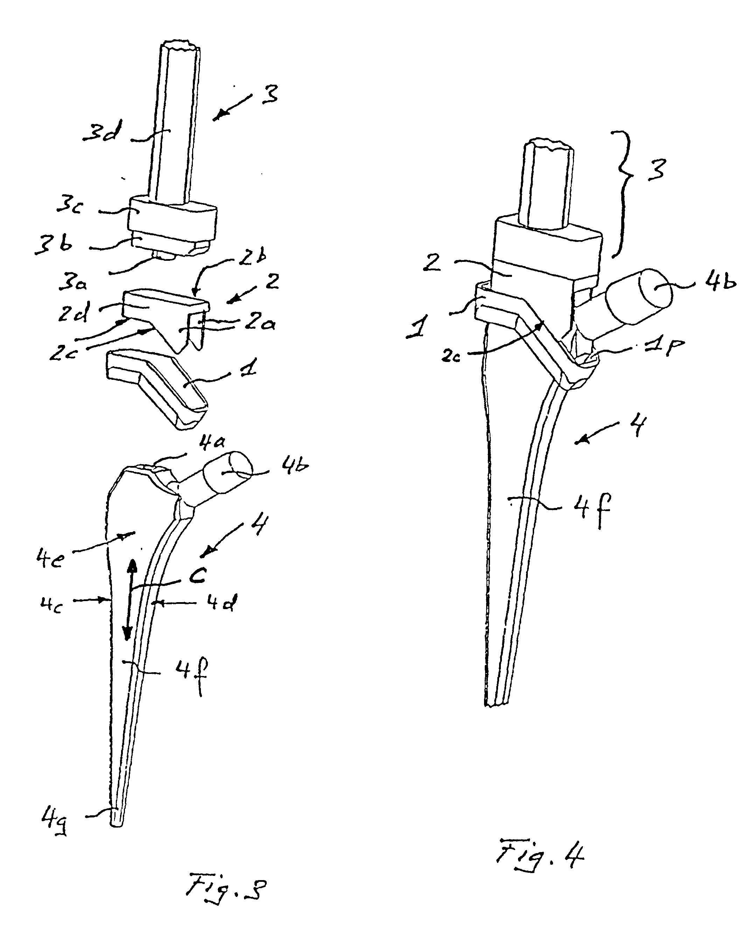

Die Endoprothese wird beispielsweise wie folgt implantiert:
Der Schenkelhals wird reseziert. Daraufhin wird der Markraum mit einer Knochenraspel erweitert. Vorzugsweise entspricht die Aussenform des Zentrierelementes, d.h. die Aussenflächen 1r,1k, der Aussenform der Knochenraspel. Draufhin wird ein Manipulierschaft in den erweiterten Markraum eingeführt und auf diesen Manipulierschaft ein Gelenkkopf aufgesetzt. Allenfalls kann auch die Knochenraspel zur Aufnahme eines Gelenkkopfes ausgestaltet sein oder einen Gelenkkopf umfassen und daher vorerst im Markraum belassen werden. Daraufhin wird die Lage des Gelenkkopfes überprüft und das Bein manipuliert, um Beispielsweise die Beinlänge zu überprüfen, wobei die Setztiefe des Manipulierschaftes bzw. der Knochenraspel insbesondere in proximal-distaler Richtung C verstellt werden kann, bis eine für das Bein optimale Lage gefunden wird. Daraufhin wird die Setztiefe des Manipulierschaftes bzw. der Knochenraspel abgelesen. Daraufhin wird der Manipulierschaft bzw. die Knochenraspel aus dem Markraum entfernt, vorauf der Knochenzement in den Markraum eingefüllt wird. Daraufhin wird, wie in Fig. 6 dargestellt, das Zentrier- und / oder Dichtelement 1 über die Spitze des Schaftes 4f geschoben, der Schaft am Setzinstrument 3 befestigt, gegebenenfalls unter Verwendung von die Setztiefe bestimmenden Distanzelementen 2, wie in Fig. 3 oder Fig. 4 dargestellt, worauf der Schaft in den Markraum eingeführt wird. Das Zentrier- und / oder Dichtelement 1 wird ebenfalls in den Markraum eingeführt. Dabei unterstützt der sich verjüngende Teilabschnitt 1l ein sicheres und zentriertes Einführen des Zentrier- und / oder Dichtelementes 1 in den Markraum. Das Zentrier- und / oder Dichtelement 1 wird derart hineingedrückt, dass die Abschlussflächen 1m, 1n vorzugsweise bündig zur Resektionsfläche verlaufen, voraufhin der Schaft weiter eingeführt wird, bis die vorbestimmte Setztiefe erreicht ist. Daraufhin wird das Setzinstrument 3 und das gegebenenfalls verwendete Distanzelement 2 entfernt.

Im dargestellten Ausführungsbeispiel ist, wie aus Fig. 2c ersichtlich, die schmalseitige Innenseitenfläche 1d in proximal-distaler Richtung C verlaufend ausgestaltet, was den Vorteil aufweist, dass diese Fläche beim Einführen des Schaftes 4f als Anlage- und Referenzfläche dient, wobei diese Fläche 1d kein Verschieben des Schaftes 4f in medial-lateraler Richtung A verursacht.

An der Schaftspitze 4g kann zudem ein distales Zentrierelement angeordnet sein.

Zum Erzielen einer Dichtwirkung ist es erforderlich, dass die Innenseitenflächen 1b,1c,1d,1e des Zentrier- und / oder Dichtelementes 1 entsprechend der Geometrie des Schaftes 4f angepasst verlaufend ausgestaltet sind, um eine Dichtwirkung zu erzielen. Daher können diese Innenseitenflächen 1b,1c,1d,1e, vorgegeben durch die Form des entsprechenden Schaftes 4f, auf unterschiedlichste Weise verlaufend ausgestaltet sein, derart, dass eine Dichtwirkung zwischen Schaft 4f und Zentrier- und / oder Dichtelement 1 erzielt wird, bei gleichzeitig gegenseitiger Verschiebbarkeit in proximal-distaler Richtung C.

Fig. 6 zeigt ein Ausführungsbeispiel eines Bausatzes, welcher einen Endoprothesenschaft 4, ein distales Zentrier- und / oder Dichtelement 1 sowie ein Setzinstrument 3 umfasst. Am Schaft 4 sind Markierungen 6 angebracht, welche die Setztiefe mit den Zahlen "0", "5" und "10" markieren. Das Setzinstrument 3 weist ebenfalls Markierungen 6 mit denselben Zahlen auf. Während dem Implantieren wird vorerst mit einem Manipulierinstrument die optimale Setztiefe ermittelt, wobei am Manipulierinstrument Markierungen sowie Zahlen angebracht sind. Die bei der Resektionsfläche liegende Markierung wird abgelesen. Daraufhin wird das Zentrier- und / oder Dichtelement mit dessen Oberkante 1m, 1n bündig zur Resektionsfläche im Markraum verankert und der Knochenzement in den Markraum eingeführt. Daraufhin wird der Schaft 4 am Setzinstrument 3 befestigt und in den Markraum eingeführt. Während dem Einführen kann der Operateur über die Markierungen 6 die Einführtiefe des Schaftes 4 bezüglich der Oberkante 1m, 1n des Zentrier- und / oder Dichtelementes genau bestimmen.

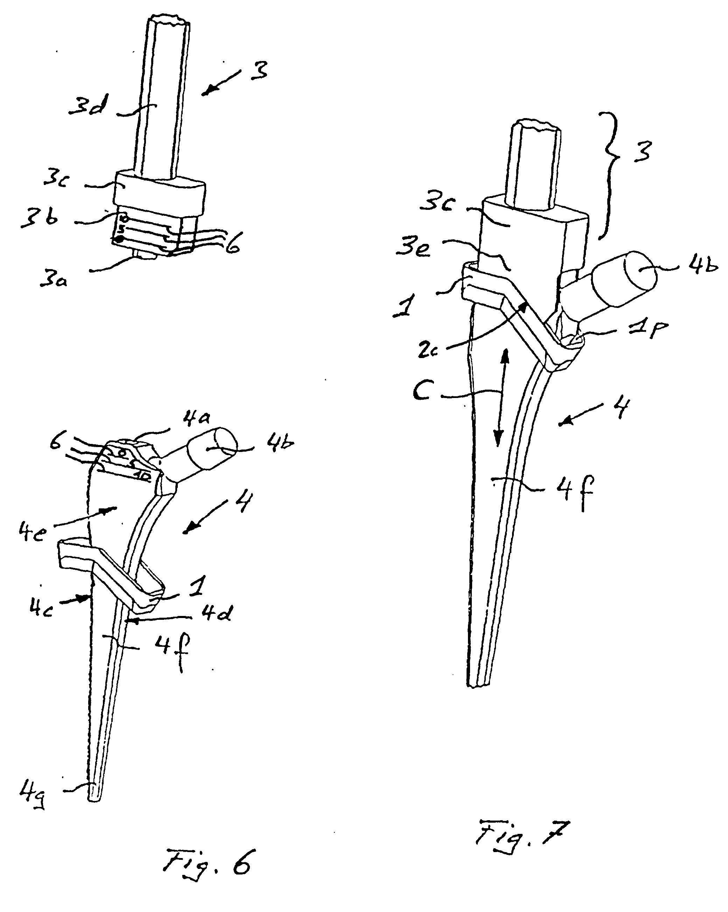

Fig. 7 zeigt ein weiteres Ausführungsbeispiel eines Bausatzes, welcher einen Endoprothesenschaft 4, ein distales Zentrier- und / oder Dichtelement 1 sowie ein Setzinstrument 3 umfasst. Der Stempel 3c umfasst ein fest verbundenes Anschlagteil 3e, welches eine Anschlagfläche 2c aufweist, zum Anschlag an das Zentrier- und Distanzelement 1. Es können Stempel 3c mit in Richtung C unterschiedlich lang ausgestalteten Anschlagteile 3e vorgesehen sein, so dass die gegenseitige Lage von Zentrier- und / oder Dichtelement 1 sowie Schaft 4 über die entsprechende Wahl des Stempels 3c einstellbar ist.

## Patentansprüche

1. Bausatz zum Implantieren einer zementfixierbaren Endoprothese, umfassend zumindest zwei zu implantierende Komponenten, einen Endoprothesenschaft (4) und ein proximales Zentrier- und / oder Dichtelement (1), wobei das Zentrier- und / oder Dichtelement (1) am Schaft (4) anlegbar und in dessen Verlaufsrichtung verschiebbar ausgestaltet ist, **dadurch gekennzeichnet, daß** der Bausatz ein Setzinstrument (3) umfaßt, wobei der Schaft (4) sowie das Setzinstrument (3) gegenseitig ankoppelbar ausgestaltet sind, und wobei entweder das Setzinstrument (3) ein Anschlagteil (2,3e) umfasst, welches einen Anschlag bezüglich dem Zentrier- und / oder Dichtelement (1) ausbildet, oder am Setzinstrument (3) beziehungsweise am Schaft (4) eine Markierung (6) angebracht ist, um eine definierte gegenseitige Lage zwischen dem Zentrier- und / oder Dichtelement (1) sowie dem Schaft (4) zu gewährleisten.

2. Bausatz nach Anspruch 1, **dadurch gekennzeichnet, dass** das Anschlagteil (3e) als separates Distanzelement (2) ausgestaltet ist.

3. Bausatz nach Anspruch 2, **dadurch gekennzeichnet, dass** das Distanzelement (2) als hülsenförmiger Körper (2d) ausgestaltet ist.

4. Bausatz nach Anspruch 2 oder 3, **dadurch gekennzeichnet, dass** das Distanzelement (2) als vorzugsweise rechteckförmiger Körper (2d) ausgestaltet ist, dessen breitseitige Begrenzungen (2a) einen zum Zentrier- und / oder Dichtelement (1) hin ausgerichteten Anschlag (2c) ausbilden.

5. Bausatz nach einem der Ansprüche 2 bis 4, **dadurch gekennzeichnet, dass** mehrere Distanzelemente (2) vorgesehen sind, welche in Verlaufsrichtung des Schaftes (4) unterschiedlich lang ausgestaltet sind.

6. Bausatz nach Anspruch 1, **dadurch gekennzeichnet, dass** das Anschlagteil (3e) ein Bestandteil des Setzinstrumentes (3) ist, und insbesondere mit diesem fest verbunden ist.

7. Bausatz nach einem der vorhergehenden Ansprüche, **dadurch gekennzeichnet, dass** das Zentrier- und / oder Dichtelement aus einem hülsenförmigen, sich in einer proximal-distalen Richtung (C ) erstreckenden Körper (1) besteht, und dass der Körper (1) einen derart ausgestalteten Innenraum (1a) mit zum Schaft (4f) weisenden Begrenzungen (1b,1c,1d,1e) aufweist, dass zumindest drei der am Schaft (4f) anliegenden Begrenzungen (1b,1c,1d,1e) eine in proximal-distaler Richtung ( C) dichtende Wirkung ausüben.

8. Bausatz nach einem der vorhergehenden Ansprüche, **dadurch gekennzeichnet, dass** das Zentrier- und / oder Dichtelement aus einem hülsenförmigen, sich in einer proximal-distalen Richtung (C ) erstreckenden Körper (1) besteht, dass der Körper (1) zwei breitseitige Begrenzungen (1q,1r) sowie zwei schmalseitige Begrenzungen (1f,1g) umfasst, welche einen im wesentlichen rechteckförmigen Innenraum (1a) umschliessen, und dass die zwei breitseitigen Begrenzungen (1q,1r) je eine Innenseitenfläche (1b,1c) ausbilden, welche in proximal-distaler Richtung ( C) im wesentlichen parallel verlaufen.

9. Bausatz nach Anspruch 8, **dadurch gekennzeichnet, dass** zumindest die breitseitigen Begrenzungen (1q,1r) einen sich in proximal-distaler Richtung (C )erstreckenden Teilabschnitt (1l) mit einer sich zur distalen Richtung hin verjüngenden Wandstärke ausbilden.

10. Bausatz nach Anspruch 7 bis 9, **dadurch gekennzeichnet, dass** zumindest die breitseitigen Begrenzungen (1q,1r) je eine in proximaler Richtung weisende Abschlussfläche aufweisen, welche eine Referenzfläche (1m,1n) ausbildet.

11. Bausatz nach Anspruch 10, **dadurch gekennzeichnet, dass** die Referenzfläche (1m,1n) im medial-lateraler Richtung (A) geknickt verlaufend ausgestaltet ist.

12. Bausatz nach einem der Ansprüche 7 bis 11, **dadurch gekennzeichnet, dass** das Element (1) aus einem verfestigen Knochenzement, insbesondere Polymethylmetacrylat (PMMA), oder aus Metall, insbesondere einer Titanverbindung, besteht.

13. Bausatz nach einem der Ansprüche 7 bis 12, **dadurch gekennzeichnet, dass** die mediale, schmalseitige Begrenzung (1g) eine in proximal-distaler Richtung (C) verlaufende Innenseitenfläche (1d) und/oder ein in proximal-distaler Richtung (C) verlaufender Spalt (1o) aufweist.

14. Bausatz nach einem der Ansprüche 7 bis 13, **dadurch gekennzeichnet, dass** die breitseitigen Innenseitenflächen (1b,1c) in distaler Richtung leicht konvergierend verlaufen.

15. Bausatz nach einem der vorhergehenden Ansprüche, umfassend ein Manipulierinstrument, welches zum Einführen in den Markraum des Femurs ausgestaltet ist, und welches die Einführtiefe des Schaftes (4) in den Markraum zu bestimmen erlaubt.

16. Bausatz nach Anspruch 15, **dadurch gekennzeichnet, dass** das Manipulierinstrument einen Schaft mit derart angebrachten Markierungen aufweist, dass bei in den Femur eingeführtem Manipulierinstrumentenschaft die Einführtiefe bezüglich der Resektionsfläche des Femurs ablesbar ist.

17. Bausatz nach Anspruch 15 oder 16, **dadurch gekennzeichnet, dass** das Manipulierinstrument eine Knochenraspel umfasst.

## Claims

1. A kit for implanting a cementable endoprosthesis, comprising a fitting instrument (3) and at least two components to be implanted, namely an endoprosthesis shaft (4) and a proximal centering and/or sealing element (1), the shaft (4) and the fitting instrument (3) being designed to be able to be coupled to each other, and the centering and/or sealing element (1) being designed to be placeable on the shaft (4) and to be displaceable in the direction of extension of the latter, and either the fitting instrument (3) comprising a limit stop part (3e) which forms a limit stop relative to the centering and/or sealing element (1), or a marking (6) being arranged on the fitting instrument (3) and on the shaft (4) in order to ensure a defined mutual position between the centering and/or sealing element (1) and the shaft (4).

2. The kit as claimed in claim 1, wherein the limit stop part (3e) is designed as a separate spacer element (2).

3. The kit as claimed in claim 2, wherein the spacer element (2) is designed as a sleeve-shaped body (2d).

4. The kit as claimed in claim 2 or 3, wherein the spacer element (2) is designed as a preferably rectangular body (2d) whose broad-side boundaries (2a) form a limit stop (2c) oriented toward the centering and/or sealing element (1).

5. The kit as claimed in one of claims 2 through 4, wherein a plurality of spacer elements (2) are provided which are designed with different lengths in the direction of extension of the shaft (4).

6. The kit as claimed in claim 1, wherein the limit stop part (3e) is a constituent part of the fitting instrument (3), and in particular is connected securely thereto.

7. The kit as claimed in one of the preceding claims, wherein the centering and/or sealing element consists of a sleeve-shaped body (1) extending in a proximal-distal direction (C), and wherein the body (1) has an inner space (1a) with boundaries (1b, 1c, 1d, 1e) pointing toward the shaft (4) designed in such a way that at least three of the boundaries (1b, 1c, 1d, 1e) bearing on the shaft (4f) exert a sealing effect in the proximal-distal direction (C).

8. The kit as claimed in one of the preceding claims, wherein the centering and/or sealing element consists of a sleeve-shaped body (1) extending in a proximal-distal direction (C), wherein the body (1) comprises two broad-side boundaries (1q, 1r) and two narrow-side boundaries (1f, 1g) which enclose an essentially rectangular inner space (1a), and wherein the two broad-side boundaries (1q, 1r) each form an inner side surface (1b, 1c) extending essentially parallel in the proximal-distal direction (C).

9. The kit as claimed in claim 8, wherein at least the broad-side boundaries (1q, 1r) form a part section (1l) which extends in the proximal-distal direction (C) and which has a wall thickness tapering in the distal direction.

10. The kit as claimed in claims 7 through 9, wherein at least the broad-side boundaries (1q, 1r) each have an end face pointing in the proximal direction and forming a reference surface (1m, 1n).

11. The kit as claimed in claim 10, wherein the reference surface (1m, 1n) is designed with a bend in the medial-lateral direction (A).

12. The kit as claimed in one of claims 7 through 11, wherein the element (1) consists of a compacted bone cement, in particular polymethyl methacrylate (PMMA), or of metal, in particular a titanium compound.

13. The kit as claimed in one of claims 7 through 12, wherein the medial, narrow-side boundary (1g) has an inner side surface (1d) extending in the proximal-distal direction (C) and/or a gap (1o) extending in the proximal-distal direction (C).

14. The kit as claimed in one of claims 7 through 13, wherein the broad-side inner side surfaces (1b, 1c) converge slightly in the distal direction.

15. The kit as claimed in one of the preceding claims, comprising a manipulating instrument which is designed for insertion into the medullary cavity of the femur and which makes it possible to determine the depth of insertion of the shaft (4) into the medullary cavity.

16. The kit as claimed in claim 15, wherein the manipulating instrument has a shaft with markings arranged thereon in such a way that when the manipulating instrument shaft is inserted into the femur, the depth of insertion can be read off relative to the resected surface of the femur.

17. The kit as claimed in claim 15 or 16, wherein the manipulating instrument comprises a bone rasp.

## Revendications

1. Jeu de composants pour l'implantation d'une endoprothèse cimentable, comprenant au moins deux composants à implanter, à savoir une tige d'endoprothèse (4) et un élément proximal de centrage et/ou d'étanchéité (1), l'élément de centrage et/ou d'étanchéité (1) pouvant être appliqué sur la tige (4) et étant réalisé déplaçable dans la direction de son extension, **caractérisé en ce que** le jeu de composants comprend un instrument de mise en place (3), la tige (4) ainsi que l'instrument de mise en place (3) étant réalisés de manière à pouvoir être accouplés mutuellement, et soit l'instrument de mise en place (3) comprend une partie de butée (2, 3e) qui forme une butée par rapport à l'élément de centrage et/ou d'étanchéité (1), soit au moins une marque (6) est appliquée sur l'élément de mise en place (3) ou sur la tige (4) pour assurer une position réciproque définie entre l'élément de centrage et/ou d'étanchéité (1) et la tige (4).

2. Jeu de composants selon la revendication 1, **caractérisé en ce que** la partie de butée (3e) est réalisée sous forme d'élément d'écartement (2) séparé.

3. Jeu de composants selon la revendication 2, **caractérisé en ce que** l'élément d'écartement (2) est réalisé sous forme de corps (2d) en forme de douille.

4. Jeu de composants selon l'une ou l'autre des revendications 2 et 3, **caractérisé en ce que** l'élément d'écartement (2) est réalisé sous forme de corps (2d) de préférence rectangulaire dont les délimitations (2a) sur le côté large forment une butée (2c) orientée vers l'élément de centrage et/ou d'étanchéité (1).

5. Jeu de composants selon l'une quelconque des revendications 2 à 4, **caractérisé en ce qu'**il est prévu plusieurs éléments d'écartement (2) qui sont réalisés de différentes longueurs en direction d'extension de la tige (4).

6. Jeu de composants selon la revendication 1, **caractérisé en ce que** la partie de butée (3e) fait partie de l'instrument de mise en place (3) et est en particulier reliée de manière solidaire à celui-ci.

7. Jeu de composants selon l'une quelconque des revendications précédentes, **caractérisé en ce que** l'élément de centrage et/ou d'étanchéité (1) est constitué par un corps (1) en forme de douille qui s'étend dans une direction proximale-distale (C), et **en ce que** le corps (1) présente un espace intérieur (1a) réalisé avec des délimitations (1b, 1c, 1d, 1e) tournées vers la tige (4f) de telle sorte qu'au moins trois délimitations (1b, 1c, 1d, 1e) appliquées sur la tige (4f) exercent un effet d'étanchéité en direction proximale-distale (C).

8. Jeu de composants selon l'une quelconque des revendications précédentes, **caractérisé en ce que** l'élément de centrage et/ou d'étanchéité (1) est constitué par un corps (1) en forme de douille qui s'étend dans une direction proximale-distale (C), **en ce que** le corps (1) comprend deux délimitations (1q, 1r) sur le côté large ainsi que deux délimitations (1f, 1g) sur le petit côté, lesquelles entourent un espace intérieur (1a) sensiblement rectangulaire, et **en ce que** les deux délimitations (1q, 1r) sur le côté large forment des surfaces latérales intérieures respectives (1b, 1c) qui s'étendent sensiblement parallèles en direction proximale-distale (C).

9. Jeu de composants selon la revendication 8, **caractérisé en ce qu'**au moins les délimitations (1q, 1r) sur le côté large forment un tronçon partiel (1l) s'étendant en direction proximale-distale (C) avec une épaisseur de paroi qui se rétrécit en direction distale.

10. Jeu de composants selon l'une des revendications 7 à 9, **caractérisé en ce qu'**au moins les délimitations (1q, 1r) sur le côté large forment chacune une surface terminale tournée en direction proximale, qui constitue une surface de référence (1m, 1n).

11. Jeu de composants selon la revendication 10, **caractérisé en ce que** la surface de référence (1m, 1n) est réalisée en s'étendant de manière coudée en direction médiale-latérale(A).

12. Jeu de composants selon l'une quelconque des revendications 7 à 11, **caractérisé en ce que** l'élément (1) est constitué par un ciment osseux solidifié, en particulier par un polyméthylmétacrylate (PMMA), ou par du métal, en particulier par un composé de titane.

13. Jeu de composants selon l'une quelconque des revendications 7 à 12, **caractérisé en ce que** la délimitation (1g) médiale sur le petit côté présente une surface latérale intérieure (1d) s'étendant en direction proximale-distale (C) et/ou une fente (1o) s'étendant en direction proximale-distale (C).

14. Jeu de composants selon l'une quelconque des revendications 7 à 13, **caractérisé en ce que** les surfaces latérales (1b, 1c) du côté large convergent légèrement en direction distale.

15. Jeu de composants selon l'une quelconque des revendications précédentes, comprenant un instrument de manipulation qui est réalisé pour pouvoir être introduit dans le canal médullaire du fémur et qui permet de déterminer la profondeur d'introduction de la tige (4) dans le canal médullaire.

16. Jeu de composants selon la revendication 15, **caractérisé en ce que** l'instrument de manipulation présente une tige avec des marques appliquées de telle sorte que lorsque l'instrument de manipulation est introduit dans le fémur, la profondeur d'introduction par rapport à la surface de résection du fémur est lisible.

17. Jeu de composants selon l'une ou l'autre des revendications 15 et 16, **caractérisé en ce que** l'instrument de manipulation comprend une râpe à os.
